# EUROPEAN PATENT APPLICATION

(11) **EP 2 856 987 A1**
(43) Date of publication of application: **08.04.2015**
(21) Application number: 13187608.8
(22) Date of filing: 07.10.2013
(51) Int. Cl.: A61F 13/00

(54) **A wound dressing and a method for manufacturing a wound dressing**

(71) Applicant: Absorbest AB, 590 39 Kisa (SE)
(72) Inventor: Rovaniemi, Rolf, 590 46 Rimforsa (SE)
(74) Representative: WSL Patentanwälte Partnerschaft mbB

(57) **Abstract**

The present invention relates to a wound dressing comprising an absorbent core, a facing layer, and a backing layer, wherein the absorbent core is located between the facing layer and the backing layer, and wherein the wound dressing further comprises an antimicrobial substance. It turned out that the amount of an antimicrobial substance which is transported from these wound dressings into the wound is entirely uncontrolled and thus the antimicrobial not always fulfils its desired function. While too much of an antimicrobial substance may irritate the wound site, too little of the antimicrobial substance may have no or little effect at the wound site. It is therefore an object of the present invention to provide a wound dressing enabling an improved application of an antimicrobial substance to a wound. In order to solve this object a wound dressing is suggested comprising an absorbent core, a facing layer, and a backing layer, wherein the absorbent core is located between the backing layer and the facing layer, wherein the wound dressing comprises an antimicrobial substance, and wherein the wound dressing further comprises a barrier layer which when in use is located between a wound and the antimicrobial substance, wherein the barrier layer is arranged such that when the wound dressing is laid onto a wound the barrier layer introduces a time delay in the transport of liquid from the wound towards the antimicrobial substance.

## Description

The present invention relates to a wound dressing comprising an absorbent core, a facing layer, and a backing layer, wherein the absorbent core is located between the facing layer and the backing layer, and wherein the wound dressing further comprises an antimicrobial substance.

Furthermore the present invention relates to a method for manufacturing a wound dressing comprising the following steps: providing an absorbent core, providing a facing layer, providing a backing layer, locating the absorbent core between the facing layer and the backing layer providing an antimicrobial substance in the wound dressing, and mounting the absorbent core, the facing layer, the backing layer and the antimicrobial substance to form a wound dressing.

Wound dressings containing superabsorbent polymers in particular for application of heavily secreting wounds have been described in various forms in the prior art. For example the book "More than superabsorbent polymer technology" edited by Frederic L. Buchholz and Andrew T. Graham, published in 1998 by John Wiley and Sons, Inc., ISBN 0-471-19411-5 on page 251 discloses the usage of superabsorbent polymers for the design of effective wound dressings.

WO 03/094813 describes a wound dressing comprising a pouch, in which an absorbent core consisting of a non-woven material having superabsorbent polymers dispersed therein is located.

In order to support wound healing, wound dressings are known, which contain an antimicrobial substance somewhere in the dressing.

However, it turned out that the amount of an antimicrobial substance which is transported from these wound dressings into the wound is entirely uncontrolled and thus the antimicrobial not always fulfils its desired function. While too much of an antimicrobial substance may irritate the wound site, too little of the antimicrobial substance may have no or little effect at the wound site.

It is therefore an object of the present invention to provide a wound dressing enabling an improved application of an antimicrobial substance to a wound. It is a further object of an embodiment of the present invention to provide a wound dressing enabling a controlled application of an antimicrobial substance to a wound within a therapeutic window.

At least one of the above objects is solved by a wound dressing comprising an absorbent core, a facing layer, and a backing layer, wherein the absorbent core is located between the backing layer and the facing layer, wherein the wound dressing comprises an antimicrobial substance, and wherein the wound dressing further comprises a barrier layer which when in use is located between a wound and the antimicrobial substance, wherein the barrier layer is arranged such that when the wound dressing is laid onto a wound the barrier layer introduces a time delay in the transport of liquid from the wound towards the antimicrobial substance.

In an embodiment the absorbent core may be any structure comprising cellulose fluff, airlaid cellulose, tissue paper, a nonwoven, a textile fabric, a foam, an alginate, ALT or a hydrocolloid as an absorbent.

The absorbent core of the wound dressing in an embodiment may be any structure of a material comprising a superabsorbent substance. Superabsorbent substances in the sense of the present application are materials that have the ability to absorb and retain large volumes of water in aqueous solutions. Superabsorbent substances falling into this category are for example modified starch, polymerized polyvinyl alcohol (PVA), polyethylene oxide (PEO) which are all hydrophilic and have a high affinity to water. When chemically or physically cross-linked, these polymers are water-swellable but not water soluble. The aforementioned superabsorbent substances have been known for a long time.

In a particular embodiment of the present invention the superabsorbent substance is a superabsorbent polymer (SAP), in particular in the form of (granular) particles or fibres. In particular such a SAP is made from polymerization of acrylic acids blended with sodium hydroxide in the presence of an initiator to form a poly-acrylic acid sodium salt (sometimes referred to a sodium poly-acrylate).

In an embodiment, the absorbent core containing a SAP is formed by at least two non-woven fabric layers or at least two tissue layers or at least two cellulose layers, wherein for fabrication the SAP, preferably in the form of particles or fibres, is dispersed on the first layer, then the second layer is put on top and the two layers are consolidated providing a matrix carrying the SAP between the two layers.

A non-woven fabric in the sense of the present application is a material made of at least one layer of fibres which have been formed to a web and in the next step consolidated. In particular, consolidation of a non-woven fabric may be achieved by friction and/or cohesion and/or adhesion, for example by needling, felting, spun lacing, melting or heat embossing.

If compared to tissue paper a material will be considered a non-woven fabric in the sense of the present application once more than 50 % of the mass of its fibre components consists of fibres having a ratio of their length to their diameter of more than 300. Alternatively, the material will be considered a non-woven fabric in the sense of the present application if this condition is not fulfilled, but if more than 30 % of the mass of its fibre components consists of fibres having a ratio of their length to their diameter of more than 300 and its density is lower than 0.4 g/cm³. This is deemed to be equal to EM 29 092.

While an absorbent core as described above may be advantageous, it is not excluded to design absorbent cores using different material combinations.

An absorbent core, in particular an absorbent core having a superabsorbent substance which in the following text may also be denoted as a superabsorbent core, extracts and stores any liquid exudates from a wound, to which the wound dressing is applied to.

In order to avoid direct contact between the absorbent core and the wound surface, the wound dressing further comprises the facing layer and the backing layer.

In an embodiment the different layers of the wound dressing are stacked on top of each other and connected to each other, e.g. by gluing or heat embossing.

According to an embodiment of the present invention, a shell or cover enclosing the absorbent core may be formed by the facing layer and the backing layer, wherein the facing layer and the backing layer are joint together such that the facing layer and the backing layer form a pouch. It is evident, that the absorbent core when it is located in the pouch, is surrounded by the facing layer and the backing layer.

It is assumed that in an embodiment the facing layer and/or the backing layer is a laminar structure whose thickness is small compared to its length and width. As such the extension of the facing layer denotes its laminar extension. A direction parallel to the extension of the facing layer and/or the backing layer is essentially parallel to the plane defined by the width and the lengths of the facing layer or the backing layer. A direction perpendicular to the extension of the facing layer and/or a backing layer essentially denotes the thickness of the facing layer and/or backing layer.

In an embodiment, the facing layer and the backing layer are integrally formed from a single sheet of material. However, in some embodiments the facing layer and the backing layer are two distinct and separate layers, which enables to design their properties and functionalities individually as needed.

In order to form a pouch, the facing layer and the backing layer may be joint together by a seam, e.g. by gluing or heat embossing.

In an embodiment, the facing layer is made of a material consisting of one selected from a group consisting of a non-woven fabric, a perforated film and a foam with open cells based on polyethylene or silicone or a combination thereof.

While it may be that the facing layer is brought into direct contact with the wound, onto which the wound dressing is applied, it may be that the wound dressing in an embodiment comprises one or more further layers between the facing layer and the wound. Depending on its functionality in the wound dressing, a person skilled in the art will choose the material of the facing layer in order to fulfil such functionality.

In an embodiment, the facing layer comprises a non-woven fabric consisting of synthetic and/or cellulose fibres, wherein the fibres of a non-woven fabric are reoriented such that they predominantly extend in a direction perpendicular to the extension of the facing layer. Such a reorientation of the fibres in the non-woven fabric is achieved by orienting the fibres during the fabrication process, in particular during spun-lacing, needling or electrostatic processing.

In an embodiment of the invention, the facing layer comprises a density in a range from 0.1 g/cm³ to 0.6 g/cm³.

In a further embodiment, the facing layer in its unwetted state comprises an area weight (also denoted as the gram weight, basis weight or grammage) in a range from 12 gsm (g/m²) to 100 gsm (g/m²), preferably in a range from 18 gsm (g/m²) to 70 gsm (g/m²).

It is further useful if in an embodiment the facing layer comprises a hydrophobic or hydrophilic surface. This is in particular applicable once the facing layer is brought into direct contact with the wound.

In an embodiment of the present invention, the backing layer serves as a clothing protection. In an embodiment, the backing layer is thus advantageously made of a breathable non-woven fabric or a breathable film enabling a breathing of the wound, but preventing wound exudates from exiting the wound dressing and contaminating a patient's clothing.

While the backing layer may form the outermost layer of the wound dressing, if applicable being brought into contact with the patient's clothing, there may be embodiments, wherein there are further layers on top of the backing layer, i.e. between the backing layer and the patient's clothing, providing additional functionalities for the wound dressing.

The facing layer and the backing layer in an embodiment can be joint together in different ways. If the facing layer and the backing layer consist of an identical material, a single sheet of material can be used, folded and sealed together at its other edges in order to form the pouch.

While in an embodiment the facing layer or the backing layer may be in direct contact with the top surface and/or bottom surface of the absorbent core, there may be an embodiment, wherein an additional layer is located between the bottom surface of the absorbent core and the facing layer and/or between the top surface of the absorbent core on the backing layer.

In another embodiment the facing layer and the backing layer may both comprise a material as it has been described above with respect to the facing layer. This way either side of the wound dressing may be applied to the wound.

The wound dressing according to the present invention furthermore comprises an antimicrobial substance. While in principle the antimicrobial substance may be located anywhere in the wound dressing typically the antimicrobial substance may either be located in the facing layer, in a carrier layer between the facing layer and the absorbent core or in the absorbent core itself.

An antimicrobial substance in the sense of the present invention is any substance which is suitable in order to inactivate or kill a microorganism or to inhibit adherence and colonization or to inhibit biofilm formation or quorum sensing or to turn the microorganisms avirulent. Microorganisms are diverse. They include bacteria, fungi, virus and protozoa. In the sense of the present application all microorganisms are considered.

In an embodiment the antimicrobial substance is selected from a group consisting of silver oxide, antimicrobial polymers, in particular PHMB, quaternary ammonium compounds, in particular benzalkonium chloride, dequalinium, benzethonium chloride, cetylpyridinium chloride (CPC) and biphenyl betylpyridinium chloride, quorum sensing inhibitors, anti-inflammatory substances, chelators, e.g. EDTA, metal compounds, in particular including silver, gold, platinum, gallium, barium and zinc, iodine compounds, alcohols, diazolidinyl urea, isothiazolinone lactic acid, methylchloroisothiazolin, polyaminopropyl biguanide, potassium sorbate, propyl benzoate, sodium benzoate, nitric oxide or any combination thereof.

In an embodiment of the invention the antimicrobial substance is a dry substance. In a further embodiment the antimicrobial substance is one selected from a group consisting of a bactericide, a bacteriostatic, a fungicide or a fungistatic or a combination thereof.

Typically a wound dressing absorbs exudate, i.e. liquid containing serum proteins, inflammatory cells, microorganisms, and cell debris or slough, from a wound and stores the liquid in the absorbent core, i.e. a transport of liquid from the wound through the facing layer and into the absorbent core is effected.

In order to apply an antimicrobial substance contained in a wound dressing to the wound, the transport direction of the antimicrobial substance to be effected has to be in the opposite direction of the liquid being absorbed in the core. Such a transport may in an embodiment be provided by two effects. Liquid entering the wound dressing will dissolve the antimicrobial substance. Thus there will be a difference in concentration of the antimicrobial substance in the liquid already entered into the wound dressing and liquid outside the wound dressing, i.e. in close contact with the wound. This concentration gradient will drive the antimicrobial substance to move in the liquid from the wound dressing to the wound. On the other hand the wound dressing applied to a patient's skin, be it a human being or an animal, undergoes a steady motion due to the motion of the skin. This motion will provide some flow of liquid having already entered the wound dressing without being stored in the absorbent core from the dressing back towards the wound.

It is the basic idea of the present invention that a barrier layer is located in the wound dressing. A barrier layer may provide its functionality based on two distinct effects: On the one hand the barrier layer when being dissolved and/or passed by the liquid in an embodiment may open up contact of the wound exudate with other layers or components of the dressing containing the antimicrobial substance. On the other hand the barrier layer stops exudates from of being absorbed by the absorbent core too fast, which would prevent an effective transport of the antimicrobial substance into the wound.

In an embodiment, wherein the barrier layer is located in the wound dressing between the wound and the antimicrobial substance, the barrier layer covers the antimicrobial substance such that liquids exudating from a wound and being transported into the wound dressing towards the absorbent core will meet and interact with the barrier layer before coming into engagement with the antimicrobial substance. This interaction of liquid, in particular water, with the barrier layer on its transport path from the wound towards the absorbent core will introduce a time delay in the transport of the antimicrobial substance from the wound dressing towards the wound. By the design of the barrier layer the time delay for the transport of the antimicrobial substance from the dressing towards the wound can be set or controlled. While it is evident that the barrier layer in such an embodiment must be located on a path of the liquid from the wound towards the absorbent core, i.e. between the wound and the antimicrobial substance, there are in principle multiple arrangements how and where the barrier layer may be located, such that it protects the antimicrobial substance from immediately getting into engagement or contact with the liquid absorbed from the wound.

While in most applications it will be advantageous that the barrier layer when looked at from the wound covers or shields the antimicrobial substance, there may also be an embodiment, wherein some amount of the antimicrobial substance is not covered by the barrier layer. In a particular embodiment the antimicrobial substance is located in the facing layer or in close proximity to the facing layer and a barrier layer is located between this amount of antimicrobial substance and the absorbent core. In such an embodiment the barrier layer stops exudates from of being absorbed by the absorbent core too fast. Only exudate or liquid which is not been absorbed or trapped by the absorbent core can move freely in the wound dressing and may provide transport of the antimicrobial substance into the wound. Thus a barrier layer stopping quick absorption of the exudate in the absorbent core will hold upright a flow of antimicrobial substance into the wound.

In the following further examples of the location and design of the barrier layer are described.

In an embodiment the barrier layer comprises a liquid soluble material, preferably a water soluble material. Such a material when getting into contact with liquid being transported from the wound towards the absorbent core initially will stop the flow of liquid until the barrier layer is entirely or partly dissolved by the liquid and does not stop the liquid on its transport path anymore.

Such a barrier layer in an embodiment may comprise a polyvinyl alcohol (PVOH), a colloid, pectin, gelatine, an alginate or silicon or any combination thereof. Any of these substances will be considered water soluble in the sense of the present application. Depending on the choice of material the time the barrier layer needs to be entirely or at least partly dissolved by the liquid is different.

However, in addition to the choice of material the thickness of the barrier layer will strongly influence the time scale, which is needed for the barrier layer to be dissolved by the liquid.

In an embodiment of the invention the barrier layer has a thickness in a range from 5 µm to 150 µm, preferably in a range from 10 µm to 60 µm. The given ranges of thickness are in particular suitable, once the barrier layer comprises as film of PVOH.

In an alternative embodiment the barrier layer may be chosen to comprise a finite absorption speed for liquid. Then the time delay before the antimicrobial substance gets into contact with the liquid is determined by the time, which the liquid on its transport path from the wound towards the absorbent core needs to cross the barrier layer when first wetting the barrier layer.

In an embodiment such barrier layer based on a finite absorption speed of the barrier layer for liquid is made of tissue paper. In an embodiment tissue paper to be used as the barrier layer comprises cellulose pulp and preferably comprises 100 % virgin pulp. In an embodiment the cellulose pup has been produced chemically by breaking down those components binding the cellulose fibres together. In an embodiment a cellulose pulp chemically produced by use of the substance sulphite or sulphate processes or a combination thereof is used.

In an embodiment of the invention, the tissue paper of a barrier layer comprises an area weight in a range from 17 gsm (g/m²) to 60 gsm (g/m²), an elongation in a range from 10 % to 25 %, and a wet strength in length in a range from 60 N/m to 300 N/m.

In the sense of the present application the elongation is the extent to which a paper can be stretched starting from its original unbiased state until it breaks or rips. The elongation is expressed in percentage. If for example the original length of a piece paper is 100 mm and the paper has an elongation of 20 % then the paper will be ripped once the piece of paper has been stretched to a length of 120 mm.

By choosing and setting the before-mentioned parameters, i.e. the area weight, elongation and wet strength in length, the properties of the tissue paper used to form the barrier layer in an embodiment may be set such that the wet strength fits to the desired release time of the antimicrobial substance, i.e. the time between applying the wound dressing to a wound and arrival of the antimicrobial substance at the actual wound.

The wet strength in the sense of the present application is a measure of how well the fibre web of a tissue paper holds together upon a force of rupture. Wet strength here is expressed as the tensile force at break of the wet paper given in N/m.

Once a medium release time is to be achieved, in an embodiment a tissue layer may be used as a barrier layer having an area weight of 20 gsm (g/m²), an elongation length of 20 % and a wet strength of 100 N/m.

Once a slow release time is required in an embodiment a tissue paper may be used as a barrier layer having an area weight of 40 gsm (g/m²), an elongation in length of 20 % and a wet strength of 300 N/m.

For a medium release time in an embodiment a tissue paper should be chosen having a wet strength in length in a range of more than 100 N/m to 150 N/m

For a slow release time in an embodiment a tissue paper should be chosen having a wet strength in length of more than 150 N/m and up to 600 N/m.

There may be an embodiment, wherein the antimicrobial substance is included in the barrier layer itself. This would in particular be feasible once the barrier layer comprises a finite absorption speed for liquid and is for example made of tissue paper, cellulose pulp, a textile, a non-woven, an alginate or a hydrocolloid.

However, in another embodiment the barrier layer has a sheet-like form extending between the facing layer and the absorbent core and in a particular embodiment may be a film extending between the facing layer and the absorbent core. This way the barrier layer effectively introduces a time delay for any liquid passing the facing layer on its transport path from the facing layer towards the absorbent core.

In the sense of the present application a sheet-like material denotes a material whose thickness is smaller than its width and length.

While in an embodiment the antimicrobial substance may be located in the absorbent core or even in the facing layer, an embodiment may be preferred, wherein the antimicrobial substance is located in a carrier layer, wherein the carrier layer extends between the barrier layer and the absorbent core. In an embodiment this carrier layer is sheet-like or film-like as is the barrier layer.

This way in an embodiment the wound dressing may comprise a sandwich structure: when looked at from the wound comprising at least a facing layer, a barrier layer, a carrier layer with the antimicrobial substance, an absorbent core and a backing layer.

In an embodiment the carrier layer for the antimicrobial substance can be made of a non-woven material or a textile material based on anyone of a material chosen from a group consisting of rayon, cellulose, cotton or a mixture of any of these materials with any of a material from a group formed of PP, PET, polyamide or PE or a combination thereof. In a further embodiment the carrier layer consists of ALT, alginate, hydrocolloid, tissue paper or a textile material. In an embodiment the carrier layer for the antimicrobial substance may have an area weight in a range from 20 gsm (g/m²) to 150 gsm (g/m²).

It is apparent that a carrier layer for the antimicrobial substance in an embodiment may well serve as an absorbent layer itself.

In an embodiment the carrier layer containing the antimicrobial substance comprises a first sub-layer and a second sublayer, wherein when producing the carrier layer containing the antimicrobial substance, the antimicrobial substance is dispersed on the first sublayer, than the second sublayer is put on top and the two sublayers are consolidated forming an confinement for the antimicrobial substance.

In a particular embodiment the carrier layer may comprise a non-woven material being a binder-free, hydroentangled non-woven. A particular type of non-wovens fulfilling these requirements is available under the trade names "fibrella" or "biolace" from Suominen Corporation of Finland. Particular non-woven materials are suitable as a carrier layer for PHMB.

In another embodiment the carrier layer may comprise a so-called high loft non-woven material having an area weight in a range from 15 gsm (g/m²) to 150 gsm (g/m²), preferably in a range from 20 gsm (g/m²) to 80 gsm (g/m²). Such a high loft non-woven is for example commercially available from Libeltex Lt. of Meulebeke, Belgium.

In an embodiment the area of the barrier layer is smaller than the area of the absorbent core.

In a particular embodiment comprising a carrier layer containing the antimicrobial substance the area of the carrier layer and the area of the barrier layer are smaller than the area of the absorbent core. In such an embodiment it may be advantageous once the area of the carrier layer and the area of the barrier layer are essentially identical.

In an embodiment the area of the barrier layer and optionally the area of the carrier layer is by 50 %, preferably by 60 % and most preferably by 75 % smaller than the area of the absorbent core.

In an embodiment, wherein the barrier layer and optionally the carrier layer have an area being smaller than the area covered by the absorbent core, the barrier layer and optionally the carrier layer are located in the centre of the wound dressing, i.e. covering the centre of mass of the absorbent core.

In an embodiment, wherein the barrier layer and optionally the carrier layer have an area being smaller than the area of the absorbent core most of the exudate entering the wound dressing will be transported outside the area covered by the barrier layer. This may enhance effective transport of the antimicrobial substance towards the wound surface in those areas covered by the barrier layer and optionally by the carrier layer.

In particular in an embodiment, wherein the barrier layer and optionally the carrier layer cover an area being smaller than the area of the absorbent core it may be practical if the absorbent core comprises two or more sub-layers, wherein at least a first sub-layer provides a transport of exudate preliminary in a direction parallel to the lateral extension of the absorbent core, and wherein at least a second sub-layer provides a transport of exudate preliminary in a direction perpendicular to the lateral extension of the absorbent core. In an embodiment the first sub layer is located closer to the wound surface than the second sub-layer of the absorbent core.

Embodiments are feasible, wherein the wound dressing contains a single sheet-like barrier layer and a single sheet-like carrier layer with an antimicrobial substance.

However, in an embodiment of the invention the wound dressing may comprise a plurality of sheet-like barrier layers and a plurality of sheet-like carrier layers. Such an embodiment is advantageous as it provides a possibility to design a wound dressing releasing different concentrations of the antimicrobial substance over time.

In order to achieve the right concentration of the antimicrobial substance in the wound over time the thickness and/or the material of the different barrier layers may be set or chosen as well as the concentration of the respective antimicrobial substance in the carrier layers involved. This way the release of the antimicrobial substance is effected step by step to the wound over a long period of time.

In a particular embodiment of the wound dressing the number of carrier layers containing the antimicrobial substance exceeds the number of barrier layers by one. This allows for one carrier layer being uncovered by a barrier layer in close proximity to the wound surface. In an embodiment this uncovered carrier layer may even be the facing layer itself. The first carrier layer for the antimicrobial substance will very quickly release the antimicrobial substance towards the wound.

In a particular embodiment comprising a plurality of barrier layers as well as a plurality of carrier layers the amount of antimicrobial substance contained in each individual carrier layer increases when looked at in a direction from the facing layer towards the absorbent core.

Film-like barrier layers may be provided in a raw form, which can be easily processed in highspeed in a machine for manufacturing the actual wound dressing.

In a particular design of a wound dressing according to an embodiment of the invention a plurality of barrier layers and a plurality of carrier layers is arranged such that the concentration of PHMB at the wound surface is kept in a predetermined range, wherein this predetermined range in the sense of the present application is denoted the therapeutic window.

In an embodiment the concentration of the antimicrobial substance in each individual carrier layer increases when looked at from the wound site. I.e. a first carrier layer closest to the wound site has the lowest concentration and the carrier layer closest to the absorbent core has the highest concentration of the antimicrobial substance. This may help to keep the concentration of the antimicrobial substance at the wound surface in the therapeutic window.

In yet another embodiment, wherein the wound dressing comprises at least three separate carrier layers, the first carrier layer (considered in a direction from the wound site) has a higher concentration of an antimicrobial substance than the second layer and the third carrier layer has a higher concentration of the antimicrobial substance than the second carrier layer. This way the first carrier layer may introduce an initial high level of an antimicrobial substance at the wound site shortly after the wound dressing has been applied to the wound. In an embodiment the concentration over time should still continuously remain within the therapeutic window.

In another embodiment of the present invention the barrier layer may have a ball-like shape encapsulating the antimicrobial substance. Such an antimicrobial substance encapsulated in a ball-like structure may in an embodiment even be located or included in the facing layer itself. In another embodiment an antimicrobial substance being encapsulated in a ball-like barrier layer may be included in a carrier layer having a sheet-like form and preferably extending between the facing layer and the absorbent core.

Ball-like shaped barrier layers encapsulating another substance, in case of the present invention the antimicrobial substance, are well-known from the prior art in order to release an active compound in a controlled way. Such encapsulating techniques have been described for example in international patent applications WO 2011/104410 or WO 2012/007628. Ball-like shaped barrier layers are available in different morphology: Mononuclear balls contain the antimicrobial substance in a shell surrounding the antimicrobial substance. Polynuclear ball-like barrier layers have many cores containing the antimicrobial substance enclosed within a single shell or barrier layer. Matrix encapsulation means, that the barrier layer contains multiple ball-like occlusions containing the antimicrobial substance in the barrier layer. Materials available in order to form a barrier layer having a ball-like shape are gums, carbohydrates, celluloses, lipids and proteins, wherein those materials have to be chosen such that they are liquid soluble or have a finite absorption speed for liquid.

In an embodiment the wound dressing according to the present invention is self-adhesive. For example a self-adhesive film may be applied to the backing layer or as a backing layer, which laterally extends beyond the facing layer. Those sections extending beyond the facing may be attached to a patient's skin. In a particular embodiment the wound dressing forms a plaster or boarder.

In another embodiment the wound dressing comprises an adhesive which is applied to the facing layer of the dressing, wherein the adhesive is preferably applied in a pattern on the facing layer.

A boarder in the sense of the present application is a wound dressing having an occlusive or semi-occlusive film at least partly covering the backing layer and extending beyond the facing layer such that the film can be fixed to a patient's skin.

At least one of the above objects is also solved by a method for manufacturing a wound dressing comprising the following steps: providing an absorbent core, providing a facing layer, providing a backing layer, providing an antimicrobial substance in the wound dressing, and mounting the absorbent core, the facing layer, the backing layer and the antimicrobial substance to form a wound dressing, providing a barrier layer, locating the barrier layer such when in use the barrier layer is located between a wound and the absorbent core, and arranging the barrier layer such that when in use the barrier layer introduces a time delay in the transport of liquid from the wound towards the absorbent core.

As far as aspects of the present invention have been described above regarding the wound dressing, they also apply to a process for manufacturing such wound dressing and vice versa.

Further advantages, features and applications of the present invention will be apparent from the following description of embodiments thereof as well as the figures attached.
Figure 1 shows a schematic cross-sectional view of a wound dressing according to an embodiment of the present invention.
Figure 2 shows a schematic cross-sectional view of a second embodiment of a wound dressing according to the present invention.
Figure 3 shows a schematic graph of the concentration of PHMB at the wound surface for a wound dressing of figure 2.
Figure 4 shows a schematic cross-sectional view of another embodiment of a wound dressing according to the present invention.
Figure 5 shows a schematic cross-sectional view of yet a further embodiment of a wound dressing according to the present invention.
Figure 6 shows an embodiment of a carrier layer to be used in a wound dressing according to an embodiment of the present invention.
Figure 7 shows a schematic cross-sectional view of a further embodiment of a wound dressing according to the present invention.
Figure 8 shows a schematic cross-sectional view of yet another embodiment of a wound dressing according to the present invention.
Figure 9 shows a schematic cross-sectional view of a further embodiment of a wound dressing according the present invention.

In the figures similar elements have been denoted by identical reference numbers.

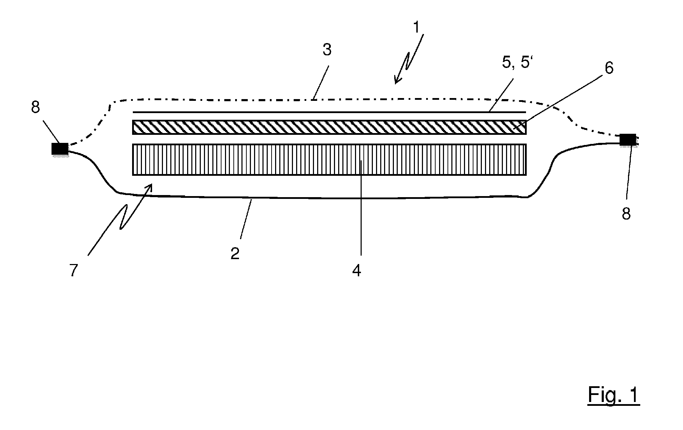

Figure 1 shows a schematic cross-sectional view of a wound dressing 1 according to an embodiment of the present invention. This wound dressing comprises a backing layer 2, a facing layer 3, an absorbent core 4, a barrier layer 5 and a carrier layer 6 containing an antimicrobial substance inside a pouch 7 formed by the backing layer 2 and the facing layer 3.

In the embodiment shown the backing layer 2 serves as the clothing protection for a patient's clothing. In order to provide this functionality the backing layer 2 is made of a breathable non-woven fabric avoiding leakage of wound exudate through the backing layer 2 onto a patient's clothing while simultaneously allowing breathing of the wound.

The facing layer 3 is thought to be brought into contact with the wound surface. There are two characteristics preferably provided by a facing layer 3 to be brought into contact with the wound surface. I.e. the facing layer 3 should be permeable for at least the liquid parts of exudate exiting a wound and the facing layer 3 should have non-sticking properties. In the example as depicted in figure 1, the facing layer 3 is thus formed by a perforated film.

When manufacturing the wound dressing 1, the backing layer 2 and the facing layer 3 are provided in separate sheet like structures and have to be joint together. The two layers 2, 3 form a pouch 7 therebetween accommodating the other components of the wound dressing 1, i.e. the absorbent core 4 as well as the barrier layer 5 and the carrier layer 6. In the present example, the backing layer 2 and the facing layer 3 are joint together by a seam 8 formed of glue.

When joint together, the backing layer 2 and the facing layer 3 form a shell or cover providing a pouch 7 as required.

In the pouch 7 an absorbent core 4 is located. This absorbent core carries a superabsorbent substance in order to provide for the required ability to store large amounts of liquid components of wound exudate extracted from the wound. In order to provide this functionality, the absorbent core 4 in the example as depicted in figure 1 is made of two sheets or layers of a non-woven fabric and poly-acrylic acid sodium salt as a superabsorbent polymer in the form of granular particles.

When manufacturing the superabsorbent core 4, the granular particles of the SAP are dispersed on a first layer of the non-woven fabric, then the second layer of the non-woven fabric is put on top such that the dispersed SAP is located between the two layers of non-woven fabric. In a last step the two layers of the non-woven fabric are consolidated such that they form a single laminar structure providing a matrix for the SAP confined between the two layers. In the particular example of figure 1 the consolidation of the two layers of non-woven fabric of the absorbent core 4 is achieved by heat embossing the two layers.

It is evident that once the wound dressing 1 is applied to a patient's skin the liquid components of wound exudate exiting the wound will be drawn towards the absorbent core 4 and the liquid will be stored by the SAP contained in the absorbent core 4.

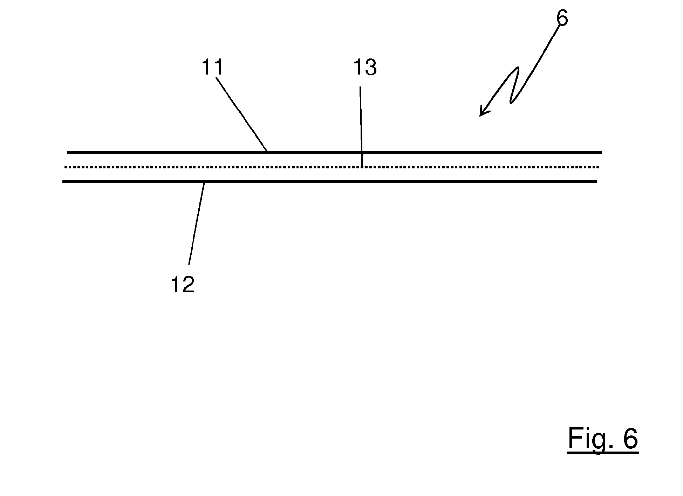

In order to prevent inflammation of the wound the wound dressing 1 of figure 1 contains PHMB as a disinfectant or antimicrobial substance. The PHMB in the present example is contained in a carrier layer 6 in a dried form, i.e. not in liquid or aqueous solution. How such a carrier layer 6 may be designed for a wound dressing 1 of figure 1 is described for example with reference to figure 6 below.

Apparently, there will be no transport or flow of PHMB from the wound dressing 1, in particular from the carrier layer 6 towards the wound as long as the carrier layer 6 is unwetted. However, the liquid components of wound exudate exiting a wound will be drawn through the facing layer 3 towards the absorbent core 4. On its transport path the liquid will pass the carrier layer 6. In the carrier layer 6 the liquid will dissolve the dried PHMB contained in the carrier layer 6.

While the dominant direction of transport of liquid is from the wound towards the absorbent core 4 in the dressing 1, a flow of PHMB dissolved in liquid has to be provided from the carrier layer 6 towards the wound and into the wound. This flow of PHMB towards the wound is established by two phenomena.

A patient's skin to which the dressing 1 is applied to is in steady motion and thus also stimulates a motion of the wound dressing itself. This motion however leads to transport of some amount of liquid with PHMB dissolved therein back to the wound. On the other hand in a first instance when liquid gets into contact with the carrier layer 6 and PHMB is dissolved in the liquid, there will be a concentration gradient between liquid in and close to the carrier layer 6 and liquid at and close to the wound surface. This concentration gradient will generate an osmotic pressure trying to equal out the concentration gradient between the barrier layer 6 and the wound surface. PHMB is effectively transported in liquid solution from the barrier layer 6 towards the wound and the transport process will continuously equal out the initial concentration gradient.

It is apparent from the above description that transport of PHMB in aqueous solution will immediately begin once liquid exudates from the wound get in contact and enter the barrier layer 6.

However, there may be situations, wherein an immediate onset of transport of PHMB from the wound dressing 1, in particular from the carrier layer 6 into the wound after application of the dressing 1 is not desirable.

In order to provide a time delay between application of the wound dressing 1 onto the wound and the onset of effective transport of PHMB into the wound, the wound dressing 1 of figure 1 comprises a barrier layer 5 which in an initial situation interrupts the flow of wound exudate from the wound through the facing layer 3, the barrier layer 6 into the absorbent core 4. The time interval between application of the dressing 1 onto the wound and the arrival of the antimicrobial substance, e.g. PHMB, at the wound surface is denoted the release time in the sense of the present application.

In order to provide such a time delay in the release time, the barrier layer 5 of the wound dressing 1 of figure 1 is made of a water-soluble material, in this particular example of PVOH. When first getting into contact the PVOH film of the barrier layer 5 will stop and interrupt the flow liquid. However, then the water contained in the exudate will dissolve the PVOH and the flow of liquid will begin to start again once the PVOH film has at least partly been dissolved by the aqueous parts of the exudate. While the choice of material for the barrier layer 5 strongly influences the time scale over which the barrier layer 5 withstands the flow of exudate, this time scale will also be influenced by the thickness of the barrier layer 5. In the present example the barrier layer 5 is made of a film having 40 µm thickness which is commercially available from MonoSol Ltd. of Merrillville, Indiana, USA. This 40 µm thick PVOH film will provide a medium release time barrier layer, i.e. a barrier layer providing a release time on an intermediate time scale.

In an alternative embodiment the barrier layer 5 is replaced by a barrier layer 5', while the overall design of the wound dressing 1 remains as it has been described with respect to figure 1 with respect to an embodiment having a barrier layer 5 comprising PVOH.

The functionality of the barrier layer 5 of the first embodiment described with reference to figure 1 is based on the effect that the onset of PHMB being dissolved in the aqueous parts of wound exudate is controlled by the time the aqueous compounds of the exudate need to dissolve the barrier layer 5 of PVOH. In the second example, the barrier layer 5' comprises a finite absorption speed for liquid. I.e. the barrier layer 5' is not dissolved by the exudate but the time delay is introduced by the fact that the exudate's transport through the barrier layer 5' is delayed due to the fact that absorption of the barrier layer 5' is comparatively slow.

In order to provide a slow release layer, the barrier layer 5' is made of tissue paper based on 100 % virgin cellulose pulp which has been produced by chemically breaking down the binder of the cellulose fibres in the initial wood by 100 % sulphite. The barrier layer 5' has an area weight of 40 gsm, an elongation length of 20 % and a resulting wet strength of 30 %.

The design of the wound dressing 1 of figure 1 is comparatively simple and allows for introducing a time delay between applying the wound dressing 1 to the wound and the onset of transport of PHMB into the wound. However, after a while the concentration of PHMB at the wound surface will reach a steady state condition and does not change anymore.

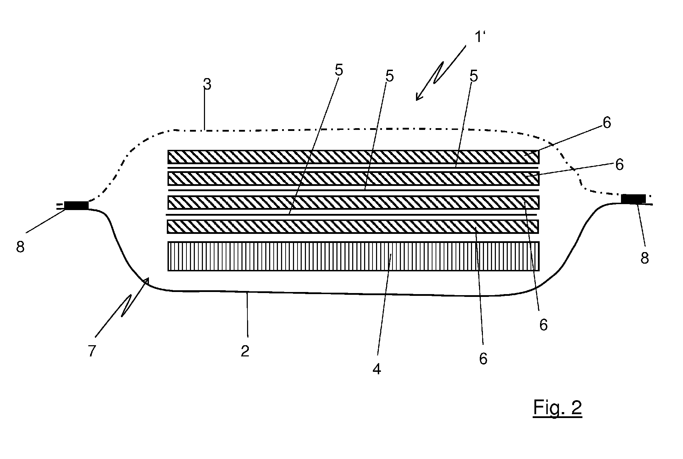

A more sophisticated design of a wound dressing 1' is depicted in figure 2. As before the wound dressing comprises a backing layer 2 and a facing layer 3 which are joint together by a seam 8 in order to form a pouch 7.

The backing layer 2 as well as the facing layer 3 may have the same design as the facing layer 2 and backing layer 3 of the dressing 1 of figure 1. Also the design of the individual layers in the pouch 7, i.e. the absorbent core 4, the carrier layers 6 and the barrier layers 5 is as described above with reference to the wound dressing 1 of figure 1. However, instead of a single barrier layer 5 and a single carrier layer 6 the wound dressing 1' of figure 2 comprises multiple carrier layers 6 and multiple barrier layers 5. In the example of figure 2 four carrier layers 6 and three barrier layers 5 are provided. This arrangement will provide a concentration of PHMB at the wound surface over time as it may be required for effective treatment of a wound.

A first carrier layer 6 is located adjacent to the facing layer 3 and is not separated from the facing layer 3 by a barrier layer. Thus, liquid compounds of exudate exiting the wound passing the facing layer 3 will immediately wetten the first carrier layer 6. PHMB is immediately dissolved such that PHMB will be provided in the right amount to the wound surface on a very short time scale. When exiting the carrier layer 6 in a direction towards the absorbent core 4 the liquid will get into contact with a first barrier layer 5. In a first instance the barrier layer 5 will interrupt the flow of liquid towards the absorbent core 4 until the barrier layer 5 is at least partly dissolved by the liquid and the transport of liquid starts again. On its path towards the absorbent core 4 the liquid will then interact with a second carrier layer 6 where it again starts dissolving PHMB which is transported towards the wound. This pattern of a barrier layer 5 stopping the transport of liquid towards the absorbent core 4 and a carrier layer 6 is then repeated twice before the liquid finally hits the absorbent core 4. Depending on the thickness of the PVOH barrier layers 5 and the concentration of the PHMB in the carrier layers 6, different concentration profiles of PHMB at the wound surface may be achieved. In fact the number and type of layers 5, 6 will be designed such that a certain required concentration pattern over time at the wound surface is fulfilled.

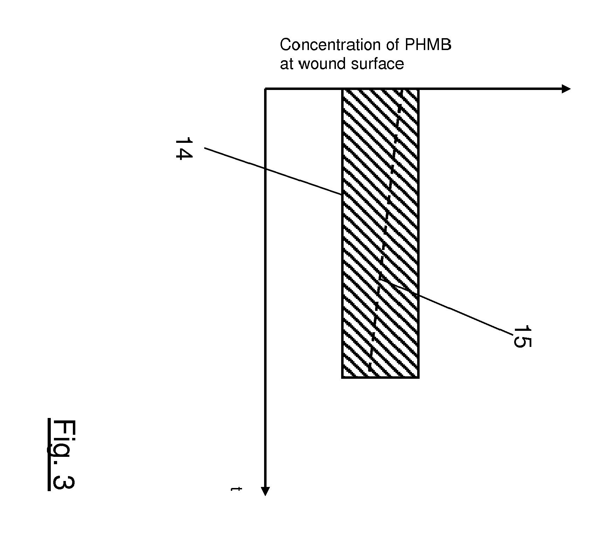

In figure 3 an example of concentration of PHMB at the wound surface over time is shown for an exemplary embodiment of the wound dressing 1' of figure 2. While on the x-axis time is plotted, the y-axis denotes the concentration of PHMB at the wound surface. It is the aim of the wound dressing 1' of Figure 2 to effect a concentration of PHMB at the wound surface lying in a certain therapeutic window 14 which is denoted by the dashed box in Figure 3. While figure 3 explicitly shows the concentration of PHMB at a wound surface the same principle applies for any other antimicrobial substance contained in a wound dressing according to the present invention.

From figure 3 it can be seen that in the wound dressing 1' of figure 2 the barrier layers 5 have approximately the same effect in time delay as they are identical layers. However, the concentration of PHMB in the carrier layers 6 is different. While the first carrier layer 6 has a considerably low concentration , the concentration of PHMB increases in the subsequent carrier layers 6 towards the absorbent core 4. Consequently the concentration of PHMB in the respective carrier layer 6 increases the closer the carrier layer 6 is located to the absorbent core 4.

As simultaneously the transport path for the PHMB from the carrier layer 6 to the wound surface increases the closer a carrier layer 6 is located to the absorbent core 4, this leads to an effective concentration of PHMB at the wound surface denoted by the dotted line 15 in figure 3 which over the time remains in the therapeutic window. Hence from a medical point of view the actual concentration of PHMB at the wound surface is considered constant as it doesn't leave the therapeutic window. What is plotted in figure 3 is an idealized view on the actual concentration. It is apparent that in real life the concentration at the wound surface cannot be raised or lowered immediately, such that the sharp onset of the graph 15 will smoothened.

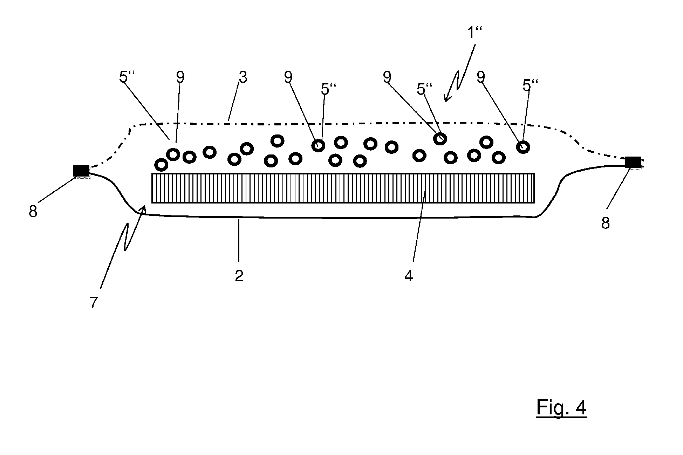

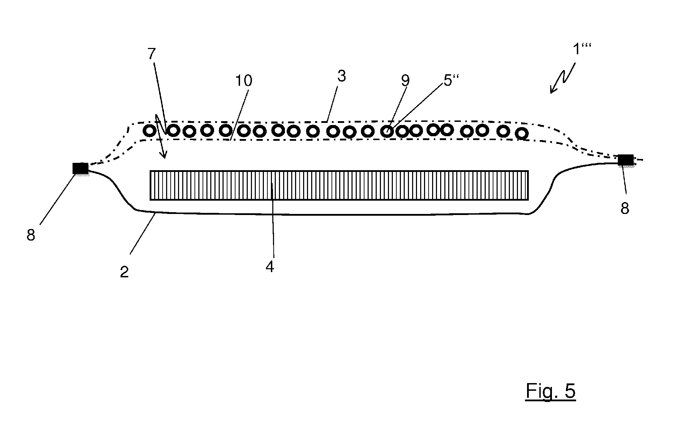

While the embodiments described with reference to figures 1 to 3 are based on laminar or film like barrier layers 5, 5', the two embodiments depicted in figures 4 and 5 are based on barrier layers 5" being ball-shaped.

The wound dressing 1" of figure 4 as before comprises a backing layer 2 and a facing layer 3 being joint together by way of a seam 8 in order to form a pouch 7, wherein in the pouch 7 an absorbent core 4 is located.

The dressing 1" of figure 4 lacks a distinct carrier layer for the PHMB. Instead the PHMB is confined in ball-shaped barrier layers 5" which are based on water-soluble material. The ball-shaped barrier layers 5" carry the PHMB 9 inside. In the present example, the barrier layers 5" are made of water-soluble gum and the ball-shaped barrier layers 5" have been coated on top of the absorbent core 4 (the latter of which is not apparent from the schematic drawing of Figure 4).

The wound dressing 1" of figure 4 with respect to its functionality as provided is fairly similar to the embodiment of figure 1 as it provides a time delay between the application of the wound dressing 1" to a wound and the onset of an effective transport of PHMB towards the wound surface. I.e. a delay in release time is achieved.

The same is basically the case for the wound dressing 1'" of figure 5 wherein however the ball-shaped barrier layers 5" are confined close to the facing layer 3 in order to be close to the wound such that the path length for the PHMB dissolved in aqueous solution to the wound surface is reduced. Thus the ball-shaped barrier layers 5" carrying the PHMB 9 are confined between the actual facing layer 3 and a confinement layer 10 being consolidated with the facing layer 3 after the ball-shaped barrier layers 5" carrying the PHMB 9 have been dispersed on the facing layer 3.

Figure 6 shows a schematic cross-sectional view of a carrier layer 6 carrying PHMB 13 inside. Production of the carrier layer 6 has some similarities with the production of the absorbent core 4 of the embodiments described with reference to figures 1, 2, 4 and 5. The actual carrier layer 6 is formed by two layers 11, 12 of a non-woven fabric. When manufacturing PHMB 13 is dispersed on the first layer 11 thereof, then the second layer 12 is put on top and the two layers 11, 12 are consolidated by heat embossing in order to form a single carrier layer 6 carrying the PHMB 13 in a matrix of a non-woven material. Alternative materials for the two sub-layers 11, 12 of the carrier layer 6 are ALT, alginate, hydrocolloid, tissue paper or a textile material.

ALT in the sense of the present application is a so-called airlaid material, which is manufactured of short cellulose fibres or a mixture of cellulose fibres and synthetic fibres. During manufacturing a web is formed of the dried fibres by means of air and afterwards a binding process is carried out to consolidate the web. During this binding process a binder, e.g. latex or an acrylic binder, both on basis of water as a solvent, is sprayed onto the web and the web is then dried to get rid of the solvent. Binding alternatively could be effected by heat bonding once synthetic fibres are used to form the web.

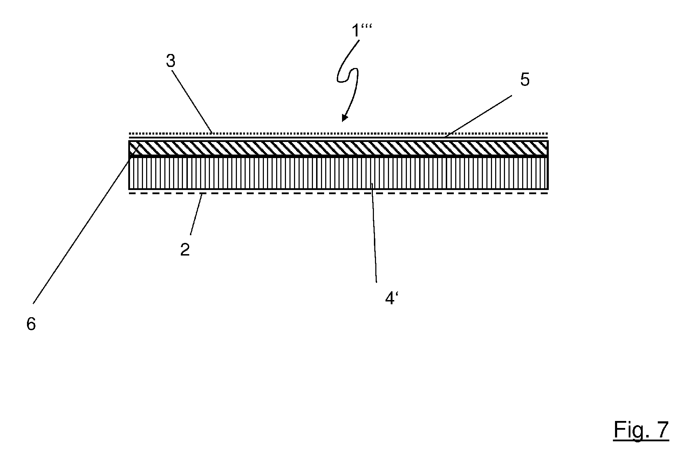

Figure 7 shows a further embodiment of a wound dressing 1'" according to the present invention. Like the dressing 1 of figure 1 the dressing 1'" comprises a facing layer 3, a backing layer 2, an absorbent core 4', a barrier layer 5, and a carrier layer 6. Consequently the dressing 1'" provides the same functionality as the dressing 1 of figure 1, when it comes to the barrier layer 5 and the carrier layer 6. However, other than the dressing of figure 1 the absorbent core 4' does not contain any superabsorbent substance but is an absorbent core 4' comprising alginate. Furthermore the facing layer 3 and the backing layer 2 are not joint together by a seam. Thus they do not form a pouch. All five layers 2, 3, 4', 5, 6 are laminated on top of each other in order to provide monolithic structure not requiring any pouch.

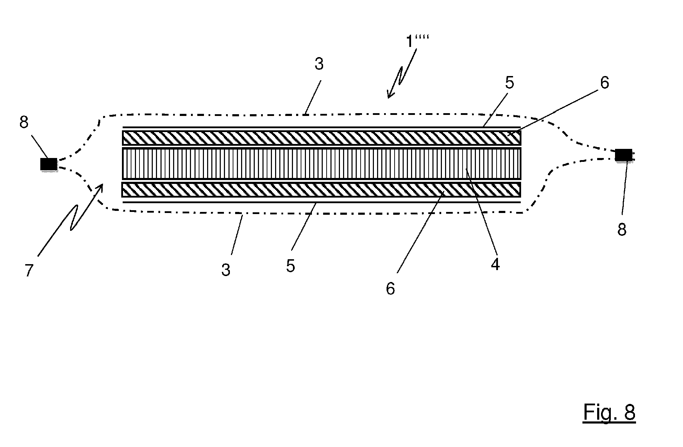

The wound dressing 1"" of Figure 8 is very similar to the dressing 1 of Figure 1. However the backing layer and the facing layer do comprise the same material as the facing layer 3 of Figure 1. Thus the facing layer and the backing layer of Figure 8 both have been denoted by reference numbers 3. This way the dressing 1"" can be applied to a patient's skin either with the facing layer 3 or the backing layer 3 facing towards the wound. I order to provide the functionality of the barrier layer 5 and the carrier layer 6 for both ways of application the dressing 1"" contains a barrier layer 5 and a carrier layer 6 on both sides of the absorbent core 4.

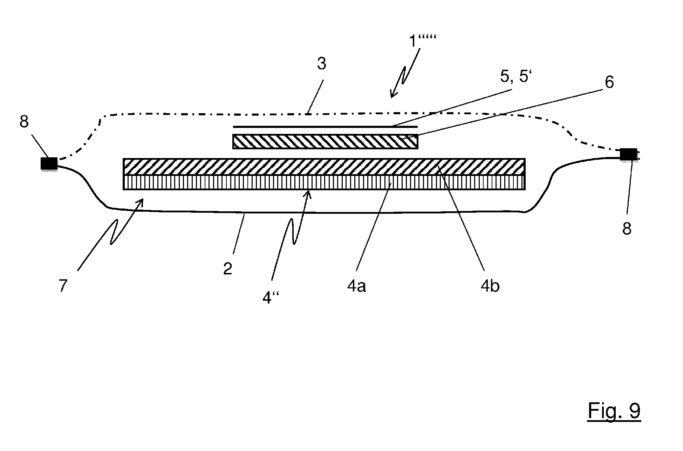

In the embodiments according to figures 1, 2, 7, and 8 the area covered by the barrier layer(s) 5, 5" and the carrier layer(s) 6 are generally identical as is the area covered by the absorbent core 4" . In contrast in the embodiment according to figure 9 the area covered by the barrier layer 5 and the carrier layer 6 (the barrier layer and the carrier layer are identical in size) is by approximately 75 % smaller than the area of the absorbent core 4".

Thus most transport of exudate from the wound surface to the absorbent core 4" is effected in those areas of the wound dressing, wherein the absorbent core 4" is not shielded by the barrier layer 5 and carrier layer 6. This may enhance the effective transport of the antimicrobial substance, in the given example PHMB, towards the wound.

In order to distribute the exudate more or less homogenously over the total area of the absorbent core 4" and thus to use the absorptive properties of the absorbent core 4" to their full extend, the absorbent core 4" comprises two sub-layers 4a and 4b. The first sub-layer 4a mainly serves to store the exudate and thus provides a transport of exudate in a direction essentially perpendicular to the lateral extension of the absorbent core 4". The second sub-layer 4b mainly serves as a distribution layer, distributing the exudate in a direction essentially parallel to the lateral extension of the absorbent core 4". Thus the second sub-layer 4b provides for a transport of exudate predominantly in a direction parallel to the lateral extension of the absorbent core 4".

For purposes of original disclosure it is pointed out that all features which are apparent for a person skilled in the art from the present application, the figures and the claims, even if they have only been described with further features, could be combined in their own or together with all the combinations of the features disclosed herein, if not excluded explicitly or technically impossible. A comprehensive explicit description of all possible combinations of the features is only omitted in order to provide readability of the description.

### Reference signs list

- 1, 1', 1'', 1''', 1'''', 1''''': wound dressing
- 2: backing layer
- 3: facing layer
- 4, 4', 4'': absorbent core
- 4a: first sub-layer (storage layer)
- 4b: second sub-layer (distribution layer)
- 5, 5', 5'': barrier layer
- 6: carrier layer
- 7: pouch
- 8: seam
- 9: PHMB
- 10: confinement layer
- 11: first layer
- 12: second layer
- 13: PHMB
- 14: therapeutic window
- 15: effective concentration of PHMB at the wound surface

## Claims

1. A wound dressing (1, 1', 1 ") comprising
an absorbent core (4),
a facing layer (3),
a backing layer (2), and
an antimicrobial substance,
**characterized in that**
the wound dressing (1, 1', 1") further comprises a barrier layer (5, 5', 5") when in use being arranged between a wound and the absorbent core (4), wherein the barrier layer (5, 5', 5") is arranged such that when in use the barrier layer (5, 5', 5") introduces a time delay in the transport of liquid from the wound towards the absorbent core (4).

2. A wound dressing (1, 1', 1") according to claim 1, wherein the barrier layer (5, 5', 5") comprises a liquid soluble material, preferably a water soluble material.

3. A wound dressing (1, 1', 1") according to claim 2, wherein the barrier layer (5, 5', 5") comprises a poly vinyl alcohol (PVOH), a colloid, pectin, gelatine, an alginate or silicone or any combination thereof.

4. A wound dressing (1, 1', 1") according to claim 2 or 3, wherein the barrier layer (5, 5', 5") is a film comprising PVOH and preferably having a thickness in a range from 5 µm to 150 µm, most preferably in a range from 10 µm to 60 µm.

5. A wound dressing (1, 1', 1") according to claim 1, wherein the barrier layer (5, 5', 5") comprises a finite absorption speed for liquid.

6. A wound dressing (1, 1', 1 ") according to claim 5, wherein the barrier layer (5, 5', 5") is made of tissue paper, preferably having a wet strength in a range from 60 N/m to 300 N/m.

7. A wound dressing (1, 1', 1") according to any of the previous claims, wherein the barrier layer (5, 5', 5") has a sheet-like form extending between the facing layer (3) and the absorbent core (4) and preferably is a film extending between the facing layer (3) and the absorbent core (4).

8. A wound dressing (1, 1', 1 ") according to any of the previous claims, wherein the antimicrobial substance is located in a carrier layer (6), wherein the carrier layer (6) extends between the facing layer (3) and the absorbent core (4), and wherein preferably the carrier layer (6) is a sheet-like or web-like carrier layer.

9. A wound dressing (1, 1', 1 ") according claim 8, comprising a plurality of barrier layers (5, 5', 5") and a plurality of carrier layers (6).

10. A wound dressing (1, 1', 1 ") according to any of claims 1 to 6, wherein the barrier layer (5, 5', 5") has a ball-like shape encapsulating the antimicrobial substance.

11. A wound dressing (1, 1', 1 ") according to one of the previous claims, wherein the antimicrobial substance is included in the barrier layer (5, 5', 5").

12. A wound dressing (1, 1', 1 ") according to one of the previous claims, wherein the absorbent core (4) comprises a superabsorbent substance.

13. A wound dressing (1, 1', 1 "), according to one of the previous claims, wherein the facing layer (3) and the backing layer (2) are joint together such that the facing layer (3) and the backing layer (2) form a pouch (7), and wherein the absorbent core (4) is located in the pouch (7).

14. A wound dressing (1, 1', 1 ") according to one of the previous claims, wherein the antimicrobial substance is selected of a group consisting of silver oxide, antimicrobial polymers, in particular polyhexanide (PHMB), quaternary ammonium compounds, in particular benzalkonium chloride, dequalinium, benzethonium chloride, cetylpyridinium chloride (CPC) and biphenyl betylpyridinium chloride, quorum sensing inhibitors, anti-inflammatory substances, chelators, in particular Ethylenediaminetetraacetic acid (EDTA), metal compounds, in particular including silver, gold, platinum, gallium, barium and zinc, iodine compounds, alcohols, diazolidinyl urea, , isothiazolinone lactic acid, methylchloroisothiazolin, polyaminopropyl biguanide, potassium sorbate, propyl benzoate, sodium benzoate, nitric oxide or any combination thereof.

15. A method for manufacturing a wound dressing (1, 1', 1 ") comprising the steps providing an absorbent core (4),
providing a facing layer (3),
providing a backing layer (2),
providing an antimicrobial substance in the wound dressing (1, 1', 1"), and
mounting the absorbent core (4), the facing layer (3), the backing layer (2) and the antimicrobial substance to form a wound dressing (1, 1', 1 "),
**characterized in that** it further comprises the steps
providing a barrier layer (5, 5', 5"),
locating the barrier layer (5, 5', 5") in the wound dressing (1, 1', 1 ") such that when in use the barrier layer (5, 5', 5") is located between a wound and the absorbent core (4), and arranging the barrier layer (5, 5', 5") such that when in use the barrier layer (5, 5', 5") introduces a time delay in the transport of liquid from the wound towards the absorbent core (4).
